# EUROPEAN PATENT APPLICATION

(11) **EP 2 500 410 A1**
(43) Date of publication of application: **19.09.2012**
(21) Application number: 11158897.6
(22) Date of filing: 18.03.2011
(51) Int. Cl.: C12M 3/00, A61F 2/24, C12N 5/00

(54) **Bioreactor with mechanical and electrical stimulation means**

(71) Applicant: Ludwig-Maximilians-Universität München, 80539 München (DE)
(72) Inventor: Akra, Bassil, 81249 Munich (DE); König, Fabian Hugo, 83088 Kiefersfelden (DE)
(74) Representative: Lucke, Andreas

(57) **Abstract**

Disclosed herein is a bioreactor for creating and/or conditioning biological tissues, in particular cardiovascular patches. This bioreactor supports additionally cellular differentiation under controlled conditions. The bioreactor comprises a housing having a first section (20) for holding a nutrition solution and/or a cell suspension (24), means (28) for holding a scaffold (34), said means being disposed or disposable inside said bioreactor housing such that said scaffold (34) held therein is exposed to said nutrition solution and/or cell suspension (24) when contained in said first section (20), mechanical stimulation means (54, 68, 86) for applying mechanical stress and/or strain to said scaffold (34) and/or tissue formed thereon when held by said scaffold holding means (28) in said bioreactor housing, and electrical stimulation means (38, 92) for applying an electrical stimulation to said scaffold (34) and/or tissue formed thereon when held by said scaffold holding means (28) in said bioreactor housing.

## Description

### FIELD OF THE INVENTION

The present invention relates to the field of tissue engineering. In particular, the invention relates to a bioreactor for supporting cellular differentiation and/or creating and/or conditioning biological tissues, in particular cardiovascular patches.

### RELATED PRIOR ART

Cardiovascular diseases are the first leading cause of mortality in the western hemisphere. A cardiac infarction may occur when one or more blood vessels supplying the heart are clogged. If the blood circulation is not immediately restored, an irreversible premature death of the cells of the affected area of the heart muscle arises. Since the myocardium consists of entirely differentiated tissue, it will not regenerate and can hence not compensate the loss of cells. Instead, the patient will suffer from a cardiac insufficiency that may necessitate a cardiac transplantation. However, since the number of donor organs available is much less than the demand, alternative ways of therapy are needed, see H. Jawad et. al, Myocardial tissue engineering: a review. Journal of Tissue Engineering and Regenerative Medicine, August 27, 2007, p. 327-342*.*

A promising approach in this regard is the transplantation of stem cells occurring naturally in the body directly into the myocardium. The stem cells are typically directly injected into the ventricle wall or injected to the blood. Unfortunately, the survival rate of the cells is rather low due to the comparatively high physical stress and rapid change of environmental conditions, see M Siepe et al., Construction of skeletal myoblast-based polyurethane scaffolds for myocardial repair. Artificial Organs. February 3, 2007, p. 425-433*.*

One way to optimize this procedure and to improve the efficiency is to seed a cell carrier, referred to as "scaffold" herein, with cells to form a "myocardium patch" that can be implanted to the patient. An ideal myocardium patch would have the morphological, physiological and functional properties of a natural heart muscle. It is expected that this way, the cell loss would be considerably less as compared to the injection of individual cells. For this, three-dimensional scaffolds need to be developed and seeded in a bioreactor.

A bioreactor specifically for creating cardiovascular patches is known from DE 100 53 014 A1. This bioreactor comprises holding means for holding a scaffold to be seeded with cells and a membrane pump positioned in parallel to the scaffold. A nutrition solution is provided between the membrane pump and the scaffold. The membrane pump is connected with an infusion pump for driving the same. While operating the membrane pump, pressure pulses are applied to the scaffold with the tissue forming thereon, thereby conditioning the tissue during its growth.

WO2005/007233 A2 discloses a strategy for creating engineered tissues by in vitro growth of isolated cells on three-dimensional scaffolds under conditions that coax the cells to develop into a functional tissue. In one embodiment, isolated cardiac myocytes were seeded onto a collagen sponge scaffold. After three days of cultivation, the engineered tissue was transferred to a chamber consisting of a 100 mm glass Petri dish fitted with two carbon rods acting as stimulating electrodes connected to a commercial cardiac stimulator via platinum wires. The engineered tissue was stimulated with square pulses, 2 ms in duration at 60 beats per minute and with 5 V for five days. This electrical stimulation mimics the electrical stimulation received by cardiac muscle tissue in vivo. Instead of seeding the scaffold with cardiac myocytes, in this patent document it is also suggested that the scaffold may be seeded with stem cells, where the electrical stimulation promotes differentiation of the stem cells into cardiac myocytes.

In addition, this prior art patent document suggests that the engineered tissue may be stimulated mechanically by strain, hydrostatic pressure, direct compression, high shear force environments and the like. However, while both, mechanical and electrical stimulation are generally addressed in this document, they are regarded as different conditioning steps that are carried out at different times in different devices.

### SUMMARY OF THE INVENTION

While some progress has been made in tissue engineering, there is still need for a bioreactor and a method for creating engineered tissues in vitro that when implanted will be successfully integrated into the body from a structural and functional point of view.

A bioreactor allowing for supporting cellular differentiation and/or the creation and/or conditioning of improved biological tissues is defined in claim 1. A method for supporting cellular differentiation and/or creating and/or conditioning biological tissue is defined in claim 15. Preferable embodiments are defined in the dependent claims.

The bioreactor according to the invention comprises a housing having a first section for holding a nutrition solution and/or a cell suspension and means for holding a scaffold, said means being disposed or disposable inside said bioreactor housing such that said scaffold held therein is exposed to a nutrition solution and/or a cell suspension when contained in said first section. Further, the bioreactor comprises mechanical stimulation means for applying mechanical stress and/or strain to said scaffold and/or tissue formed thereon when held by said scaffold holding means in said bioreactor housing, and electrical stimulation means for applying electrical stimulation to said scaffold and/or tissue formed thereon when held by said scaffold holding means in said bioreactor housing.

According to the invention, the scaffold and the tissue formed thereon can be simultaneously exposed to a mechanical and electrical stimulation in one and the same bioreactor. If the bioreactor is used for growing e.g. cardiovascular patches, the physiological conditions experienced by the tissue in vivo can be mimicked with regard to both, mechanical stress and/or strain due to the contraction and expansion of the heart muscle as well as with regard to the electrical stimulation produced by the electrical conduction system of the human heart. It is believed that this way the tissue-engineered patches will acquire structural and functional characteristics that are very close to the native tissue, which in turn will allow successful implantation to the patient.

Note that in this disclosure, the term "scaffold" is to be understood in the broadest possible sense, including any type of template or carrier suitable for growing tissue thereon. Such scaffolds may have different shapes and structures, including fabric-like structures, sponge-like structures or fleece-like structures, and can be made of different materials, in particular nondegradable synthetic or degradable synthetic materials or decellularized natural scaffolds.

Preferably, the electrical stimulation means and the mechanical stimulation means are controlled or operatively coupled to be operated in a synchronized fashion. In particular, the electrical and mechanical stimulation means may be operated such that an electrical stimulation is supplied when the tissue is in a relaxed or nearly relaxed state. The relaxation phase is, where the colonized scaffold actively returns to its initial position after induced contraction. Herein, a "nearly relaxed state" may be defined as a state in which the stress and/or strain is less than 40%, preferably less than 20% of the maximum stress and/or strain applied by the mechanical stimulation means. This way, the temporal combination of mechanical and electrical stimulation of cardiac tissue can be mimicked in vitro, thereby functionalizing the tissue created in vitro to be as close as possible to native tissue.

In a preferred embodiment, the electrical stimulation means comprises at least one electrode integrated with a scaffold holding means and arranged such that the scaffold and/or tissue formed thereon is in electrical contact with said at least one electrode when the scaffold is held by said scaffold holding means. Integrating the electrode in the scaffold holding means has proven to be a very efficient way to promote the electrical stimulation and at the same time facilitate the handling of the bioreactor.

Preferably, the electrical stimulation means comprises controllable pacer means for generating electrical stimulation pulses mimicking electrical stimulation received by cardiac muscle tissue in vivo. For example, the pacer means can be controlled to generate electrical stimulation pulses at frequencies between 30 to 80 beats/min with stimulation currents in a range of 0.1 to 20 mA and with a sensitivity of 1 to 10 mV.

Likewise the bioreactor further comprises control means for controlling said mechanical stimulation means such as to apply stress and/or strain to said scaffold and/or tissue formed thereon mimicking mechanical stress and strain experienced by cardiac muscle tissue in vivo.

In a preferred embodiment, the control means comprises an input interface for inputting one or more of the following parameters: a frequency of mechanical stimulation pulses, a time profile of mechanical stimulation pulses and an amplitude of mechanical stimulation pulses. By inputting these parameters, the mechanical stress and/or strain experienced by the tissue can be adapted to selected environments corresponding to different conditions of heart beats for example faster or slower pulse beats. In a particularly advantageous embodiment, the control means comprises an input interface for inputting a virtual heart pumping yield (ml/s), that is transformed by the control means to a mechanical stimulation pattern mimicking the mechanical stress and/or strain experienced by cardiac muscle tissue at such yield. That is to say, for a given heart pumping yield, a corresponding frequency and amplitude of mechanical stimulation pulses is calculated that is close to what the true cardiac muscle tissue would experience when a heart is pumping at said yield. This allows for a better assessment of the physiological conditions imitated by the bioreactor under operation.

In a preferred embodiment, the mechanical stimulation means comprises a flexible membrane separating a second section and a third section of said bioreactor, wherein the membrane is adapted to receive a pressure and/or pressurized medium for operating the same. In a preferred embodiment, the flexible membrane is removably attached to one of the second and third sections. For example, the membrane may be held by a membrane holding means comprising upper and lower clamping members for clamping the membrane inbetween, wherein the membrane holding means is removably attached to one of said second and third sections. By operating the membrane, a pressure can be generated in the second section which in turn applies a stress and/or strain to the scaffold. Also, the membrane allows to hermetically seal the second and third section from each other, which means that the third section need not be sterilized prior to operation. Since the membrane is removably attached to one of said second and third sections, the membrane itself can be easily sterilized.

In a preferred embodiment, the mechanical stimulation means comprises a piston arranged to pressurize said third section such as to operate the membrane. Further, the mechanical stimulation means preferably comprises drive means for operating said piston, such as a linear motor. A linear motor is particularly advantageous, as it is comparatively small and compact. Also, with a linear motor, any desired piston movement can be generated, hence allowing for generating arbitrary stress and/or strain profiles at the scaffold.

Preferably, the drive means is disposed in a drive housing removably connected with said third section of the bioreactor housing.

Preferably, the bioreactor comprises a seat for receiving the scaffold holding means, where the seat is arranged such that the scaffold holding means and the scaffold held thereby separate said first and second sections of the bioreactor housing. Accordingly, a pressure generated in the second section by operation of the membrane will bend the scaffold towards the first section and thereby generate a stress similar to a stress experienced by natural cardiac muscle tissue when a heart is beating.

Preferably, the scaffold holding means comprises an annular upper holding member and an annular lower holding member, said upper and lower holding members being suitable for holding, in particular clamping a scaffold inbetween, and means for connecting said upper and lower holding members. Preferably, an electrode is integrated with one of said upper and lower holding members such as to contact the scaffold when held inbetween said holding members.

In a preferred embodiment, the bioreactor housing comprises at least one port for introducing and/or discharging said nutrition solution and/or cell suspension to and/or from at least one of said first and second sections of said bioreactor housing. Further, said first section preferably comprises at least one gas exchange port for exchanging gas with the outside atmosphere, said gas exchange port being provided with a sterile filter. By exchanging gas with the outside atmosphere, typically the atmosphere within an incubator, optimum conditions for tissue growth can be promoted in the first section.

Preferably, the bioreactor housing comprises sensor means for sensing parameters and/or the time evolution of parameters indicative of the creation and/or conditioning process, and in particular for sensing one or more of the following parameters: pressure, temperature, CO₂ concentration and pH of the nutrition solution and/or cell suspension. Based on these parameters, the creation and/or conditioning process can be optimized, thereby allowing to generate biological tissues with improved quality and stability.

In a preferred embodiment, the first section of the bioreactor housing comprises at least one optical access port allowing optical access to the biological tissue inside the bioreactor housing. In the simplest case, the optical access portion can be a suitable window allowing visual inspection of the biological tissue by the naked eye. Preferably, however, the optical access portion is adapted to receive an optical instrument such as an endoscope or a CCD camera, thereby allowing a careful and high resolution inspection of the (stimulated) tissue and optionally also a recording and/or documentation of the images. By closely optically inspecting the progress of the tissue creation and/or conditioning, the operation of the bioreactor can be suitably adapted such as to obtain a biological tissue of improved quality. Note that the optical access portion allows only optical access, but no physical access to the inside of the bioreactor housing. Accordingly, the sterility inside the bioreactor housing is not compromised. Also, this means that the optical instruments such as the endoscope or CCD camera do not have to be sterilized prior and after inspection, which greatly adds to the convenience of the operation of the bioreactor.

In a preferred embodiment, the bioreactor housing consists at least in part of transparent material, and in particular of acrylic glass, glass or plastic. Due to the transparency of the material, the filling and the pH of the nutrition solution can be checked by visual inspection. A further advantage of an acrylic glass, glass or plastic housing is that it can be easily and efficiently sterilized by formaldehyde or ethylene oxide sterilization, or by steam sterilization.

In a preferred embodiment, the bioreactor housing comprises separate parts that are connectable with each other via threaded connections. This way, the separate parts of the bioreactor housing can be simply screwed together such as to assemble the bioreactor housing. This can be done most easily and conveniently and even under sterile conditions. Herein, the separate parts may comprise one or more of the following: a lower portion resembling said third section of said bioreactor housing, a middle portion resembling said second section and at least a part of said first section of said bioreactor housing, and a lid portion for closing said fist section.

### BRIEF DESCRIPTION OF THE FIGURES

- Fig. 1: is a longitudinal sectional perspective view of a bioreactor according to an embodiment of the invention,
- Fig. 2: is a cross-sectional perspective view of the bioreactor of Fig. 1,
- Fig. 3: is a sectional perspective view of a lower portion of the bioreactor housing of Fig. 1 and 2, a piston and a linear motor,
- Fig. 4: is an exploded view of a scaffold holding means, as seen from an angle below,
- Fig. 5: is an exploded view of a scaffold holding mean, as seen from an angle above, and
- Fig. 6: is a stroke-time diagram representing a mechanical stimulation pulses.

### DESCRIPTION OF A PREFERRED EMBODIMENT

For the purpose of promoting an understanding of the principle of the invention, reference will now be made to the preferred embodiment illustrated in the drawings and specific language will be used to describe the same. It will nevertheless be understood that no limitation of the scope of the invention is thereby intended, such alterations and further modifications in the illustrated device and method and such further applications of the principles of the invention as illustrated therein being contemplated therein as would normally occur now or in the future to one person skilled in the art to which the invention relates.

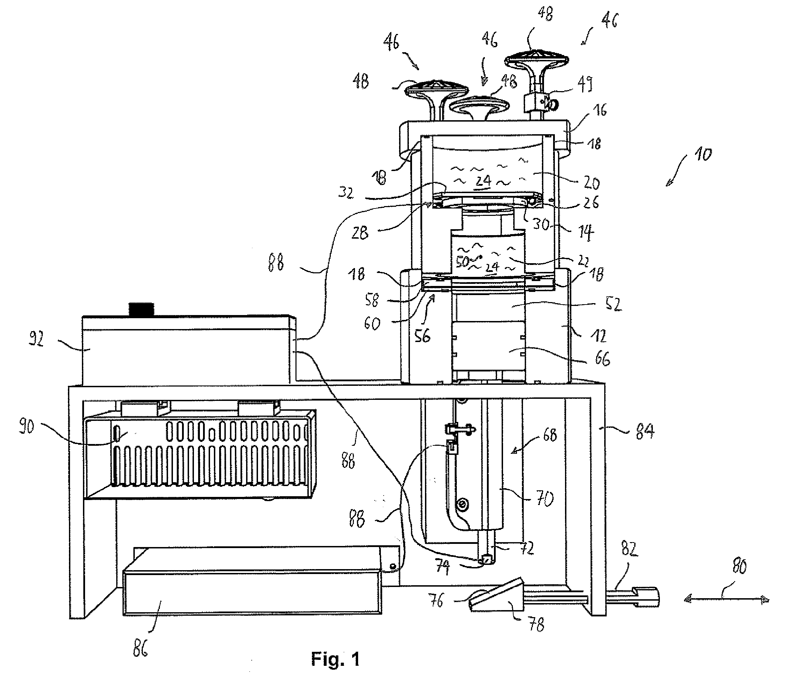

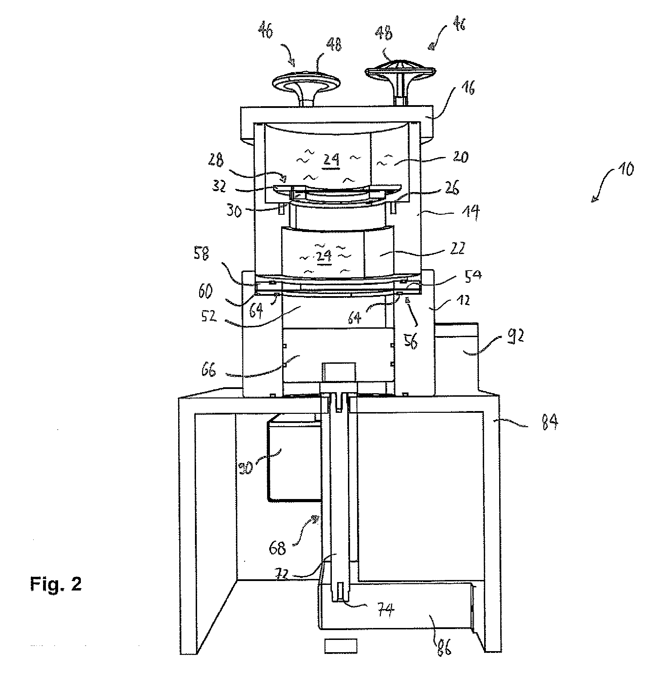

Fig. 1 shows a perspective longitudinal sectional view of a bioreactor 10. Fig. 2 shows a cross-sectional perspective view of the bioreactor 10 of Fig. 1. The bioreactor 10 has a housing comprising of a lower portion 12, a middle portion 14 and a lid portion 16. In the embodiment shown, each of the lower, middle and lid portions 12, 14, and 16 are made from acrylic glass. The lower, middle and lid portions 12, 14, and 16 are connected via threaded connections 18, such that these parts can be easily disassembled for sterilization of the individual portions and reassembled.

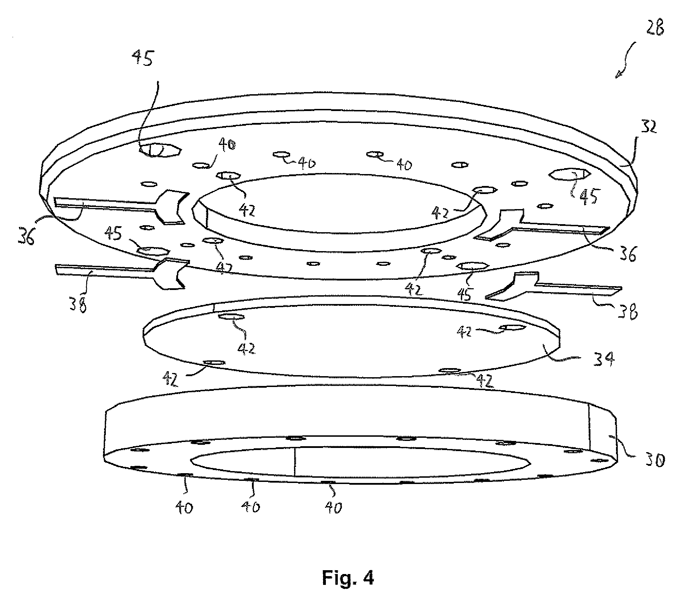

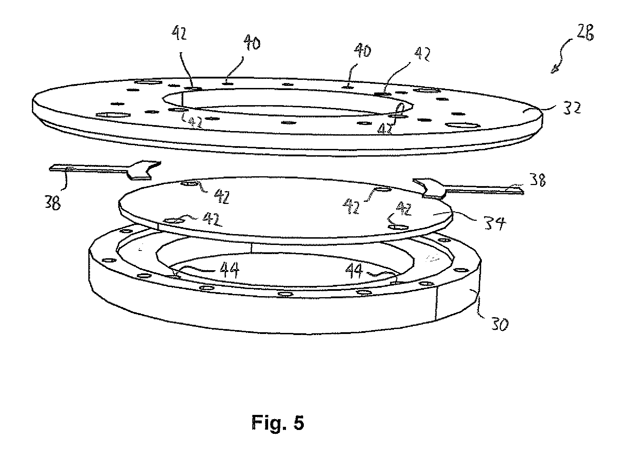

The middle portion 14 comprises a first section 20 and a second section 22. In operation, a nutrition solution and/or cell suspension 24 is contained in both, the first and second sections 20, 22. Between the first and second sections 20, 22, a seat 26 for a scaffold holding means 28 is provided, which is best seen in Fig, 2. Exploded views of the scaffold holding means 28 are further shown in Figs. 4 and 5. As is seen therein, the scaffold holding means 28 comprises a lower annular holding member 30 and an upper annular holding member 32. The upper and lower holding members 32, 30 can be attached to each other with a scaffold 34 clamped inbetween. In the present example, the scaffold 34 is of circular shape and is intended for growing a tissue engineered cardiovascular patch thereon. However, the invention is not limited to this type of scaffold and this type of application.

As is further seen in Fig. 4, recesses 36 are formed in the lower side of the upper holding member 32 for receiving electrodes 38. When the scaffold 34 is clamped between the lower and upper holding members 30, 32, the electrodes 38 are in contact with the scaffold 34 and hence with the tissue grown thereon.

A number of matching through holes 40 are provided in the lower and upper holding members 30, 32 allowing nutrition solution to pass there through and hence a restricted flow of nutrition solution/cell suspension between the first and second sections 20, 22 (see Fig. 1 and 2). Further, matching through holes 42 are formed in the upper holding member 32 and the scaffold 34. Screws (not shown) can be inserted through the through holes 42 and screwed into female threaded portions 44 provided on the upper side of the lower holding member 30, see Fig. 5.

Finally, recesses 45 for receiving spacers (not shown) are provided at the lower side of the upper holding member 32. The spacers (not shown) are meant to provide room for the electrical junctions (not shown) connected with the electrodes 38.

With reference again to Fig. 1 and 2, three gas ports 46 provided with sterile filters 48 are provided on the lid portion 16. The gas ports 46 allow for a gas exchange between the first section 20 and the outside atmosphere, i.e. typically the atmosphere within an incubator, where the bioreactor 10 would be typically disposed in operation. The gas exchange ports 46 are provided with sterile filters 48. One of the gas ports 48 is further provided with a three-way cock 49 for introducing nutrition solution and/or cell suspension to the first section 20.

A further port 50 is provided in the second section 22 for exchanging the nutrition solution/cell suspension 24 in the second section 22 if desired. The port 50 may also be used to attach a pressure sensor for measuring the pressure in the second section 22.

While not shown in the Figures, an optical access portion may be provided at the lid portion 16 allowing optical access to the scaffold 34 and the tissue grown thereon, where the optical access portion is preferably adapted to receive an optical instrument such as an endoscope or a CCD camera.

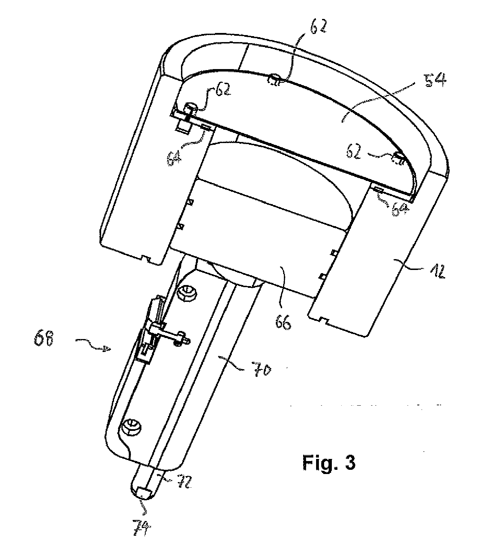

A third housing section 52 is provided in the lower portion 12. As is seen in Fig. 1 and 2, the second section 22 and the third section 52 are separated by a membrane 54 held by a membrane holding means 56 comprising upper and lower annular clamping members 58, 60 best seen in Fig. 2. The lower clamping member 60 is also shown in Fig. 3, where the upper clamping member 58 has been omitted for clarity. The upper and lower holding members 58, 60 are fastened to each other by screws 62, seen in Fig. 3, thereby clamping the membrane 54 inbetween. The membrane holding means 56 is in turn sandwiched between the middle portion 14 and lower portion 12 when the middle and lower portions 14, 12 are connected via the threaded connection 18, as is shown in Fig. 1 and 2. In this assembled state, both the second and third sections 22, 52 are hermetically sealed by o-rings 64, best seen in Fig. 2 and 3.

A piston 66 is slidingly received in the third section 52 and driven by a linear motor 68 comprising a stator 70 and a runner 72. The runner 72 can be thought of as an "unrolled" rotor of an ordinary rotary electric motor. At the lower tip of the runner 72 a gauge head 74 is provided that is formed by a simple push-button-like member in the present embodiment. The gauge head 74 is operated when it touches an abutment surface 76 of an abutment member 78 that is movable along a horizontal direction indicated by arrow 80 via a push rod 82.

The linear motor 68 and the abutment member 78 are provided in a drive housing 84. In the embodiment shown, the lower housing portion 12 is removably connected with the drive housing 84 for easy cleaning thereof. Note, however, that the lower portion 12 will generally not need to be sterilized. Instead, for guaranteeing a sterile environment of the scaffold 34 and the nutrition solution/cell suspension 24, it is sufficient that the middle portion 14, the lid portion 16, the scaffold holding means 28 and the membrane 54 and membrane holding means 56 are sterilized.

Further included in the drive housing 84 is a controller 86 for controlling the linear motor 68. The controller 86 is operatively coupled with the linear motor 68 by a signal line 88 which is only symbolically represented in Fig. 1 at reference sign 88. A power supply unit 90 is likewise contained in the drive housing 84 for supplying power to the linear motor 68 via an electrical connection (not shown).

Finally, pacer means 92 for electrically stimulating the scaffold 34 and the tissue growing thereon via the electrodes 38 is provided. The actual electrical connection between the pacer means 92 and the electrodes 38 is not shown in Fig. 1 and 2, but is represented by a symbolical signal line 88 instead for illustrative purposes. Finally, a signal line 88 for communication between the pacer means 92 and the gauge head 74 of the linear motor 68 is symbolically represented at reference sign 88.

As is particularly seen from Fig. 1 and 2, the bioreactor 10 is self-sustained in a sense that it does not need any additional periphery device for its operation. In particular, while most prior art bioreactors are operated by external pressure generating means, such as respirator pumps, the linear motor 68, controller 86 and power supply unit 90 allow to generate the operating pressure in a self-sustained way in the sense that it only needs to be connected to an ordinary electrical power source. Accordingly, the whole bioreactor 10, including the drive components contained in the drive housing 84, can be easily placed in a standard incubator.

Further, as has been explained before, the housing of the bioreactor 10 is comprised of separate elements that can be easily assembled and disassembled via threaded connections 18, thereby allowing convenient cleaning and sterilisation of the components. This also allows to limit the cleaning and sterilisation to only those parts that do come into contact with the nutrition solution/cell suspension 24 under operation.

Also, the bioreactor 10 shown in Fig. 1 and 2 has a modular design in the sense that different versions of the middle portion 14 or the lid portion 16 can be easily combined with the generic drive housing 84 and lower portion 12 via the threaded connections 18. For example, instead of the middle portion 14 shown in Fig. 1 and 2, a middle portion of different design, for example larger or smaller in size etc. could be used, as long as it matches the threaded connection 18 with the lower portion 12. Likewise, instead of the lid portion 16 shown in Fig. 1 and 2, different lid portions could be employed having fewer or more gas ports 46 or additional components, such as optical access portions or sensor means.

While not shown in the figures, it is understood that the bioreactor 10 may contain sensor means for sensing parameters and/or time revolution of parameters indicative of the creation and/or conditioning process of the tissue on the scaffold 34. In particular, the bioreactor may comprise detector means for sensing one or more of the following parameters: pressure, temperature, CO₂ concentration and pH of the nutrition solution/cell suspension 24, without being limited to these.

Next, the operation of the bioreactor 10 will be described. Before use, the middle portion 14, the lid portion 16, the scaffold holding means 28 and the membrane holding means 56, including the membrane 54 itself, are sterilized. The membrane holding means 56 and the membrane 54 are assembled and placed on top of the lower portion 12. Next, the middle portion 14 and the lower portion 12 are connected via the threaded connection 18, thereby fixing the membrane holding means 56 and hermetically sealing the second and third sections 22, 52 from each other.

The scaffold holding means 28 and the scaffold 34 are assembled and the scaffold holding means 28 is placed on a seat 26 formed at the bottom of the first section 20, see Fig. 2. When placing the scaffold holder 28 on the seat 26, the electrodes 38 are connected with leads (not shown) which are in turn connected with the pacer 92.

Next, the lid 16 is screwed onto the middle portion 14. A nutrition solution and/or a cell suspension 24 is filled into the first and second sections 20, 22, via the three-way cock 49 and optionally also via port 50. Next, the entire bioreactor 10, including the drive housing 84 is placed in an incubator kept at physiological temperature and gas atmosphere, including a CO₂ content of approximately 5%.

The linear motor 68 is operated under the control of the controller 86. By operation of the linear motor 68, the piston 66 moves up and down, thereby pressurizing the medium contained in the third section 52 (typically air) and bending the membrane 54 upward, thereby exerting a corresponding stress and/or strain to the scaffold 34 held in the scaffold holding means 28 via the nutrition solution/cell suspension 24 contained in the second section 22.

The stress and/or strain applied to the scaffold 34 is controlled such as to match or mimic mechanical stimulation of the natural tissue under physiological conditions. If the bioreactor 10 is used for producing engineered tissue for a cardiovascular patch, the linear motor 68 is operated such as to apply stress and/or strain to the scaffold and/or tissue formed thereon mimicking mechanical stress and/or strain experienced by cardiac muscle tissue in vivo. The controller 86 allows to generate any desired mechanical stimulation pattern, defined e.g. by the frequency of mechanical stimulation pulses, a time profile of the mechanical stimulation pulses and the amplitude thereof. This way, the mechanical stimulation of cardiac muscle tissue in vivo can be imitated very precisely. In particular, it is noted that a realistic stimulation will deviate considerably from a sinusoidal stimulation that would typically by achieved by using an ordinary rotary drive motor at constant rotary frequency.

The parameters characterizing the mechanical stimulation can be inputted to the controller 86 as desired via an interface (not shown). In the preferred embodiment, a virtual heart pumping yield, i.e. a certain volume per minute, can be inputted, which is a meaningful characteristic parameter from a physiological point of view. The controller 86 then automatically calculates a corresponding drive pattern of the linear motor 68 that will lead to a mechanical stress and/or strain experienced by cardiac muscle tissue at such yield.

An exemplary table summarizing stimulated heart muscle yields between 500 ml and 5000 ml/min and corresponding strokes of the runner 72 is shown below, where in the simplest approximation used, the beat frequency is not adapted to the yield, as could be the case in a more refined model.

| **Volume/Beat** | **Volume / 60 Beats** | **Stroke** |
|---|---|---|
| 83.33 ml | 5000 ml | 21.65 mm |
| 41.67 ml | 2500 ml | 10.83 mm |
| 16.67 ml | 1000 ml | 4.33 mm |
| 8.33 ml | 500 ml | 2.17 mm |

In addition to the mechanical stimulation, the scaffold 34 and the tissue growing thereon is also electrically stimulated by the pacer means 92 via the electrodes 38 integrated with the scaffold holder 28. By subjecting the engineered tissue simultaneously to mechanical and electrical stimulation, physiological conditions can be met best. When stem cells are seeded to the scaffold 34, this joint stimulation will cause the stem cells to differentiate into myocardiocyte-like cells. By applying the mechanical and electrical stimulation simultaneously in one and the same bioreactor, it is believed that the differentiation will be most successful. Also, as is seen from the above description, this can be achieved with a rather compact apparatus that is convenient and efficient to use.

To optimize the conditioning of the tissue, including the differentiation of the cells, the mechanical and electrical stimulations are preferably synchronized with each other. This may be achieved by providing for a joint control for the mechanical and electrical stimulations. However, this can also be achieved by operatively coupling the mechanical and electrical stimulation means in other suitable fashions. For example, as is shown in Fig. 1 and 2, when the runner 72 is in its lowermost position, i.e. with the piston 66 fully retracted, the gauge head 74 will abut onto the abutment surface 76 and hence be activated. This situation corresponds to the completely relaxed membrane 54, and hence relaxed scaffold 34. In vivo, the cardiac muscle tissue will be electrically stimulated after the heart muscle relaxes. Accordingly, in order to simulate the physiological conditions, detecting abutment of the gauge head 74 at the abutment surface 76 will cause the pacer means 92 to apply an electrical stimulation to the scaffold 34 and tissue grown thereon via electrode 38, in a very similar way as would occur in natural cardiac muscle tissue. Note that this way, only the mechanical stimulation needs to be controlled while the suitable electrical stimulation accompanying the mechanical stimulation will automatically be provided. Herein, the position of the abutment member 78 can be adjusted to the stroke of the runner 72 by positioning thereof via push rod 82.

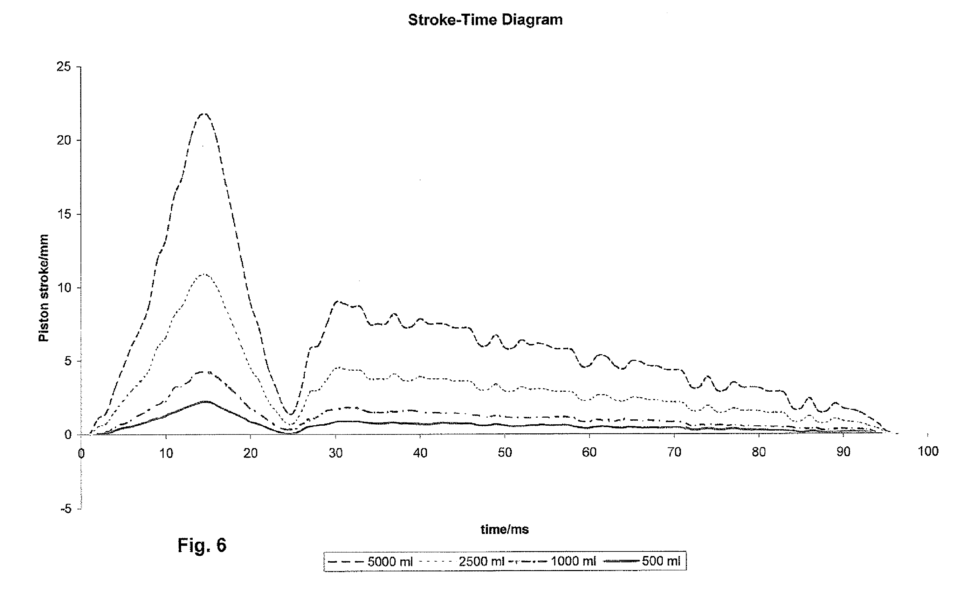

The controller 86 can control the linear motor 68 according to any desired stroke/time profile such as to generate a mechanical stimulation to the tissue that is as close as possible to physiological conditions. Exemplary profiles of stroke (mm) vs. time (ms) for one mechanical stimulation pulse are shown in Fig. 6. It is believed that in order to successfully differentiate the cells and/or create and/or condition the tissue, it is important that the mechanical stimulation is as close as possible to a stimulation as would occur in vivo. This does not only relate to the frequency and amplitude of the stimulation, which could in principle be resembled e.g. by a simple sinusoidal stimulation, but also to the proper time profile or fine structure within each mechanical stimulation pulse, as demonstrated in Fig. 6.

The embodiment described above and the accompanying figures merely serve to illustrate the apparatus and method according to the present invention, and should not be taken to indicate any limitation thereof. The scope of the patent is also determined by the following claims.

### Reference list

- 10: bioreactor
- 12: lower portion
- 14: middle portion
- 16: lid portion
- 18: threaded connection
- 20: first section
- 22: second section
- 24: nutrition solution/cell suspension
- 26: seat
- 28: scaffold holding means
- 30: lower holding member of scaffold holding means 28
- 32: upper holding member of scaffold holding means 28
- 34: scaffold
- 36: recess for receiving electrode 38
- 38: electrode
- 40: through holes
- 42: through holes
- 44: female threaded portions
- 45: recess for spacers
- 46: gas port
- 48: gas filter
- 49: three-way cock
- 50: port
- 52: third section
- 54: membrane
- 56: membrane holding means
- 58: upper holding member of membrane holding means 56
- 60: lower holding member if membrane holding means 56
- 62: screw
- 64: o-ring
- 66: piston
- 68: linear motor
- 70: stator of linear motor 68
- 72: runner of linear motor 68
- 74: gauge head
- 76: abutment surface
- 78: abutment member
- 80: arrow
- 82: push rod
- 84: drive housing
- 86: controller
- 88: signal lines
- 90: power supply unit
- 92: pacer means

## Claims

1. A bioreactor (10) for supporting cellular differentiation and/or creating and/or conditioning biological tissues, and in particular cardiovascular patches, said bioreactor (10) comprising:
- a housing having a first section (20) for holding a nutrition solution and/or a cell suspension (24),
- means (28) for holding a scaffold (34), said means (28) being disposed or disposable inside said bioreactor housing such that said scaffold (34) held therein is exposed to said nutrition solution and/or cell suspension (24) when contained in said first section (20),
- mechanical stimulation means (54, 68, 86) for applying mechanical stress and/or strain to said scaffold (34) and/or tissue formed thereon when held by said scaffold holding means (28) in said bioreactor housing, and
- electrical stimulation means (38, 92) for applying an electrical stimulation to said scaffold (34) and/or tissue formed thereon when held by said scaffold holding means (28) in said bioreactor housing.

2. The bioreactor (10) of claim 1, wherein the electrical stimulation means (38, 92) and the mechanical stimulation means (54, 68, 86) are controlled or operatively coupled to be operated in a synchronized fashion, and in particular in a fashion such that an electrical stimulation is applied when the tissue is in a relaxed or nearly relaxed state.

3. The bioreactor (10) of claim 1 or 2, wherein the electrical stimulation means (38, 92) comprises at least one electrode (38) integrated with said scaffold holding means (28) and arranged such that the scaffold (34) and/or tissue formed thereon is in electrical contact with said at least one electrode (38) when the scaffold (34) is held by said scaffold holding means (28).

4. The bioreactor (10) of one of the preceding claims, wherein said electrical stimulation means (38, 92) comprises pacer means (92) for generating electrical stimulation pulses mimicking electrical stimulation received by a cardiac muscle tissue in vivo.

5. The bioreactor (10) of one of the preceding claims, further comprising control means (86) for controlling said mechanical stimulation means (54, 68, 86) such as to apply stress and/or strain to said scaffold (34) and/or tissue formed thereon mimicking mechanical stress and/or strain experienced by cardiac muscle tissue in vivo.

6. The bioreactor (10) of claim 5, wherein said control means (86) comprises an input interface for inputting one or more of the following parameters:
- a frequency of mechanical stimulation pulses,
- a time profile of mechanical stimulation pulses,
- an amplitude of mechanical stimulation pulses,
- a virtual heart pumping yield that is transformed by the control means to a mechanical stimulation pattern mimicking the mechanical stress and/or strain experienced by cardiac muscle tissue at such yield.

7. The bioreactor (10) of one of the preceding claims, wherein said mechanical stimulation means (54, 68, 86) comprises a flexible membrane (54) separating a second section (22) and a third section (52) of said bioreactor (10), said membrane (54) being adapted to receive a pressure and/or a pressurized medium for operating the same.

8. The bioreactor (10) of claim 7, wherein said flexible membrane (54) is removably attached to one of said second and third sections (22, 52),
wherein the membrane (54) is preferably held by a membrane holding means (56) comprising upper and lower clamping members (58, 60) for clamping said membrane (54) inbetween,
wherein said membrane holding means (56) is preferably removably attached to one of said second and third sections (22, 52).

9. The bioreactor (10) of claim 7 or 8, wherein said mechanical stimulation means (54, 68, 86) comprises a piston (66) arranged to pressurize said third section (52) such as to operate the membrane (54), wherein said mechanical stimulation means (54, 68, 86) comprises drive means (68) for operating said piston (66), and in particular a linear motor (68),
wherein said drive means (68) is preferably disposed in a drive housing (84) removably connected with said third section (12) of said bioreactor housing.

10. The bioreactor (10) of one of the preceding claims, further comprising a seat (26) for receiving said scaffold holding means (28),
said seat (26) being arranged such that the scaffold holding means (28) and the scaffold (34) held thereby separate said first section (20) and a second section (22) of the bioreactor housing.

11. The bioreactor (10) of one of the preceding claims, wherein said scaffold holding means (28) comprises
- an annular upper holding member (32) and an annular lower holding member (30), said upper and lower holding members (32, 30) being suitable for holding, in particular clamping a scaffold (34) inbetween, and
- means for connecting said upper and lower holding members (32, 30),
wherein preferably at least one electrode (38) is integrated with one of said upper and lower holding members (32, 30) such as to contact the scaffold (34) when held inbetween the holding members (32, 30).

12. The bioreactor (10) of one of the preceding claims, wherein said bioreactor housing comprises at least one port (46, 50) for introducing and/or discharging said nutrition solution and/or cell suspension (24) to and from at least one of said first section (20) and a second section (22) of said bioreactor housing, and/or
wherein said first section (20) comprises at least one gas exchange port (46) for exchanging gas with the outside atmosphere, said gas exchange port (46) being provided with a sterile filter (48).

13. The bioreactor (10) of one of the preceding claims, wherein said bioreactor housing comprises sensor means for sensing parameters and/or time evolution of parameters indicative of said creation and/or conditioning process, and in particular for sensing one or more of the following parameters: pressure, temperature, CO₂ concentration and pH of the nutrition solution and/or cell suspension (24), and/or
wherein said first section (20) comprises at least one optical access portion allowing optical access to said biological tissue inside the bioreactor housing, wherein said optical access portion is preferably adapted to receive an optical instrument such as an endoscope or CCD camera.

14. The bioreactor (10) of one of the preceding claims, wherein said bioreactor housing consists at least in part of transparent material, and in particular of acrylic glass, glass or plastic, and/or
wherein the bioreactor housing comprises separate parts that are connectable with each other via a threaded connection (18), wherein said separate parts may comprises one or more of the following:
- a lower portion (12) resembling said third section (52) of said bioreactor housing,
- a middle portion (14) resembling said second section (22) and at least a part of said first section (20) of said bioreactor housing, and
- a lid portion (16).

15. A method for supporting cellular differentiation and/or creating and/or conditioning biological tissues, and in particular cardiovascular patches, comprising the following steps:
exposing a scaffold (34) to a nutrition solution and/or cell suspension (24) contained in a bioreactor housing,
applying mechanical stress and/or strain to said scaffold (34) and/or tissue formed thereon while being contained in said bioreactor housing, and
applying electrical stimulation to said scaffold (34) and/or tissue formed thereon while being contained in the same bioreactor housing,
wherein, preferably, a bioreactor (10) according to one of claims 1 to 14 is employed.
